# EUROPEAN PATENT SPECIFICATION

(11) **EP 2 429 611 B1**
(45) Date of publication and mention of the grant of the patent: **08.03.2017**
(21) Application number: 10717237.1
(22) Date of filing: 01.04.2010
(51) Int. Cl.: A61M 5/142

(54) **VALVELESS DRUG DELIVERY DEVICE**
VENTILLOSE WIRKSTOFFFREISETZUNGSVORRICHTUNG
DISPOSITIF DE DÉLIVRANCE D'UN MÉDICAMENT SANS CLAPET

(30) Priority: 07.04.2009 US 167238 P
(43) Date of publication of application: 21.03.2012
(73) Proprietor: Medimetrics Personalized Drug Delivery B.V., 5656 AE, Eindhoven (NL)
(72) Inventor: SHIMIZU, Jeff, 5656 AE Eindhoven (NL); ZOU, Hans, 5656 AE Eindhoven (NL); DIJKSMAN, Johan Frederik, 5656 AE Eindhoven (NL)
(74) Representative: Flügel Preissner Schober Seidel
(86) International application number: PCT/IB2010/051429
(87) International publication number: WO 2010/116304

(56) References cited:
- DE-A1- 3 317 536
- FR-A1- 2 390 177

## Description

### FIELD OF THE INVENTION

This invention relates to a drug delivery device comprising a drug reservoir for comprising a drug, a dispensing hole for delivering the drug to an environment of the drug delivery device and an actuator system for pushing the drug from the drug reservoir through the dispensing hole.

### BACKGROUND OF THE INVENTION

Such drug delivery devices make it possible to control the release of drugs in the gastrointestinal tract. The drug delivery may take place in accordance with a predetermined delivery schedule and/or may be triggered by external signals or environmental conditions. The device may include additional electronics, sensors and communication devices for controlling the drug delivery. The drug delivery device may either be implantable or swallowable. In many swallowable drug delivery devices, the drug release is effected using a displacement based actuator system. For example, a piston may push a predetermined amount of the drug through the delivery hole.

The dispensing hole may be an open orifice. However, an open orifice introduces the possibility for exchange of material between the reservoir and outside environment, resulting, for example, in delivery of the drug before the intended time and a reduction of the amount delivered when intended. The main processes involved are diffusion and convection. Diffusion, because of the open connection between the inside and the outside of the capsule. Convection, because of small differences in specific weight of the substance stored in the capsule and the fluid outside. E.g. when the fluid in the capsule is heavier than the fluid in the intestines and the hole is directed downwards the fluid in the capsule flows out and will be replaced by the fluid of the intestines. Both processes may be slowed down by reduction in the exit hole cross-sectional size, extending the length of the exit hole and increasing the viscosity of the material in the reservoir. However the hole size and hole length must be chosen such as to allow the actuator to dispense the material.

Often, a valve is used for separating the drug reservoir from the outside environment. Valves which operate in small spaces, provide good sealing and use low power are, however, very difficult to implement. It would thus be advantageous to avoid diffusion or convection of the drug without the use of a valve.
DE 33 17 536 A1 discloses a drug delivery device with a drug reservoir which is located in the drug delivery device in a meander-like manner. The drug reservoir is coupled to a dispensing hole by a short and straight pathway.
FR 2 390 177 describes a drug delivery device with a drug reservoir in which a solid block of a drug is stored in order to avoid accidental dispensing of the complete drug amount from the drug delivery device.

### OBJECT OF THE INVENTION

It is an object of the invention to provide a drug delivery device according to the opening paragraph, which device does not need a valve for keeping the drug inside until the intended moment of release.

### SUMMARY OF THE INVENTION

According to a first aspect of the invention, this object is achieved by providing the drug delivery device according to the opening paragraph, wherein the drug delivery device further comprises at least one exit path for coupling the drug reservoir to the dispensing hole, the exit path comprising at least one entry point for coupling the exit path to the drug reservoir, the exit path being arranged in such a way that for any possible orientation of the drug delivery device at least a part of the exit path is situated above the gravity center of said drug reservoir or below the dispensing hole.

Experiments with prototype electronic pill devices performed by the inventors show that gravity driven convection can be a dominant problem. The orientation of the capsule inside the gastrointestinal tract will vary and cannot be controlled. When the dispensing hole is facing down, i.e. towards the center of the earth, a drug with a relatively high specific weight may quickly be lost to the environment. This problem is solved with the exit path according to the invention. With the exit path according to the invention, only very small amounts of drug may pass the dispensing hole before it is intentionally pushed out of the drug delivery device. No matter what the orientation of the drug delivery device is, most of the drug must travel against gravity for travelling through the exit path and the dispensing hole. Consequently, the amount of drugs being released before the intended moment of release is very small.

Preferably, the position of the entry point relative to the dispensing hole is such that a possibility of drug release caused by a siphon effect is small. This may, e.g., be accomplished by designing the exit path in such a way that the entry point and the dispensing hole are very close together. Ifthe entry point and the dispensing hole are very close together, the gravity potential between the entry point and the dispensing hole is always small, no matter the orientation of the drug delivery device. A smaller gravity potential, results in a smaller siphon effect. Preferably, friction between the drug and the inner wall of the exit path is large enough to impede drug release due to the remaining siphon effect.

It is to be noted that the drug delivery device according to the invention, may either be implantable or swallowable. Due to movements of the user, also implantable drug delivery devices may need measures for preventing gravity driven convection.

These and other aspects of the invention are apparent from and will be elucidated with reference to the embodiments described hereinafter.

### BRIEF DESCRIPTION OF THE DRAWINGS

In the drawings:
Fig. 1 schematically shows a drug delivery device according to the invention,
Fig. 2 schematically shows an exit path for a drug delivery device according to the invention,
Fig. 3 shows a spiral exit path,
Fig. 4 shows a spiral exit path with two dispensing holes,
Fig. 5a and 5b show two different perspectives of a bottom part of an end cap with an exit path according to the invention, and
Fig. 6a and 6b show two different perspectives of a top part of the end cap of Figs. 5a and 5b.

### DETAILED DESCRIPTION OF THE INVENTION

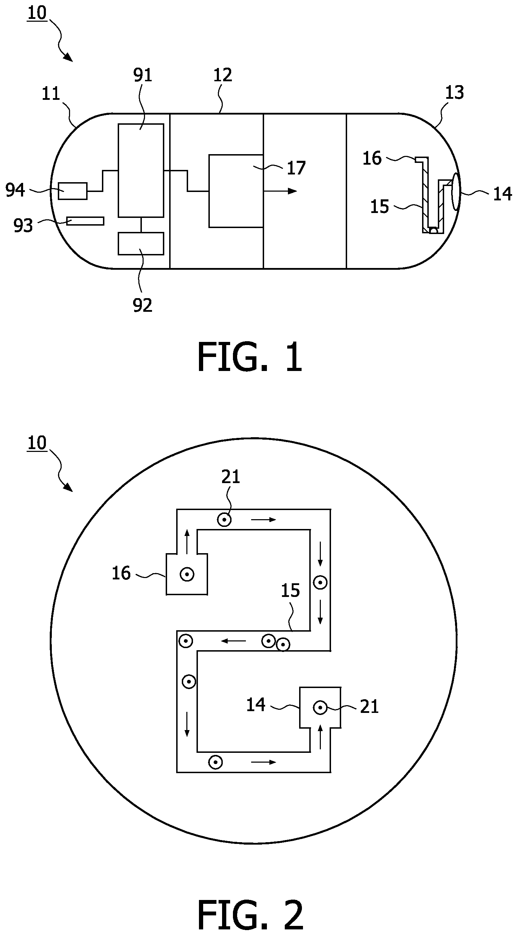

Fig. 1 schematically shows a drug delivery device 10 according to the invention. In the following, the drug delivery device 10 will be referred to as the electronic pill 10 or the pill 10. The pill 10 comprises an electronics compartment 11, an actuator compartment 12 and a drug reservoir 13. The drug reservoir 13 comprises the drug in the form of, e.g., a solution, a suspension (e.g. powder particles or microcapsules dispersed in a carrier fluid), an emulsion, a gel or a paste. The drug reservoir 13 further comprises an exit path 15 for releasing the drug into the environment of the pill 10 via the dispensing hole 14. A displacement based actuator system 17 in the actuator compartment 12 pushes the drug through an entry point 16 into the exit path 15. The actuator system 17 may, e.g., comprise a pneumatic piston or a stepper motor driven piston. The exit path 15 is arranged in such a way that for any possible orientation of the pill 10 a part of the exit path 15 is situated above gravity center of the drug reservoir or below the dispensing hole 14. The exit path 15 thereby prevents excessive loss of drug due to gravity driven convection. Exemplary designs for the exit path 15 of the pill 10 according to the invention are provided below with reference to Figs. 2 to 6.

Alternatively, the actuator system 17 may use a flexible membrane or a balloon and means for enlarging the volume of the actuator compartment 12. The volume of the actuator compartment 12 may, e.g., be enlarged using a gas produced by a chemical reaction. Preferably, the actuator system 17 is controlled by an electronic circuit 91 in the electronics department 11. The electronic circuit 91 may, e.g., initiate the drug delivery in accordance with a predetermined delivery schedule or in response to an external trigger received by a receiver 92. The electronics department 11 may further comprise one or more sensors 94 for measuring environmental conditions which may trigger the electronic circuit 91 to initiate the drug delivery. Drug release may, e.g., be triggered by specific pH values or temperatures or by the presence of specific substances, such as biomarkers. The electronics compartment 11 may comprise a transmitter 92, as a separate unit or in combination with the receiver 92, for transmitting operational data and/or sensor data to an external unit.

Fig. 2 schematically shows an exit path 15 for a drug delivery device 10 according to the invention. When the actuator 17 increases the pressure upon the drug reservoir 13, part of the drugs 21 is pushed through the entry point 16 into the exit path 15. The exit path 15 comprises multiple bends or corners which result in different sections of the exit path 15 being aligned in a different direction. Before the drugs 21 leave the pill 10 through dispensing hole 14, the drugs 21 have to travel through the complete exit path 15. Because the exit path 15 comprises different sections being aligned in different directions, there is only a very small chance that the drugs 21 traverse the complete exit path 15 due to gravity driven diffusion. For every possible orientation of the pill 10, only a small part of the exit path 15 will be aligned in such a way that gravity driven diffusion causes the drugs 21 to travel towards the dispensing hole 14. Furthermore, for every possible orientation of the pill 10, there will always be a small part of the exit path 15 which will be aligned in such a way that gravity driven diffusion causes the drugs 21 to travel away from the dispensing hole 14. Consequently, the drugs 21 will only be released into the environment when they are pushed through the complete exit path 15.

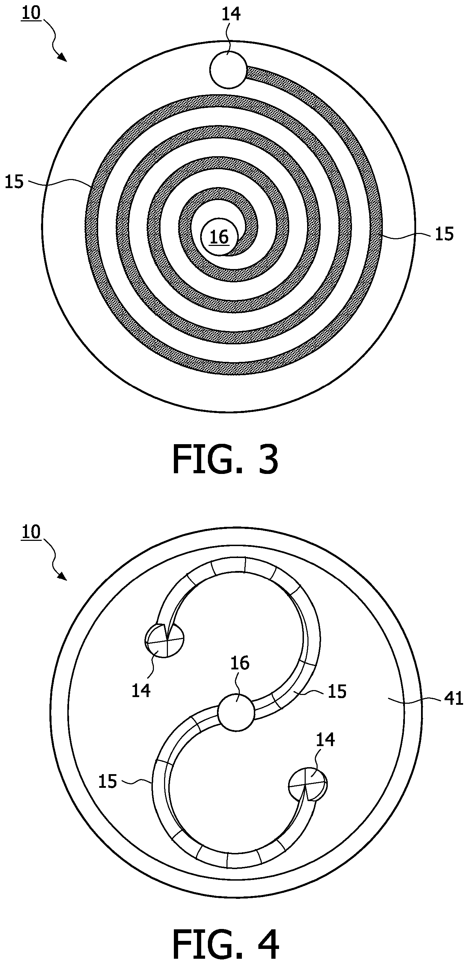

Fig. 3 shows a spiral exit path 15. The exit path 15 shown in Fig. 3 achieves the object of the invention in a way similar to the exit path 15 shown in Fig. 2.

Fig. 4 shows a spiral exit path 15 with two dispensing holes 14. The exit path 15 is integrated in an end cap 41 which closes the drug reservoir 13. The actuator system 17 pushes the drug into the exit path 15 through a central entry point 16. From the entry point 16, part of the drugs is pushed towards one dispensing hole 14 and part of the drugs is pushed towards the other dispensing hole 14. For reaching either one of the dispensing holes 14, the drugs have to travel into multiple different directions. Thus, like in Figs. 1 and 3, gravity driven convection alone will not lead to release of significant amounts of the drug into the environment.

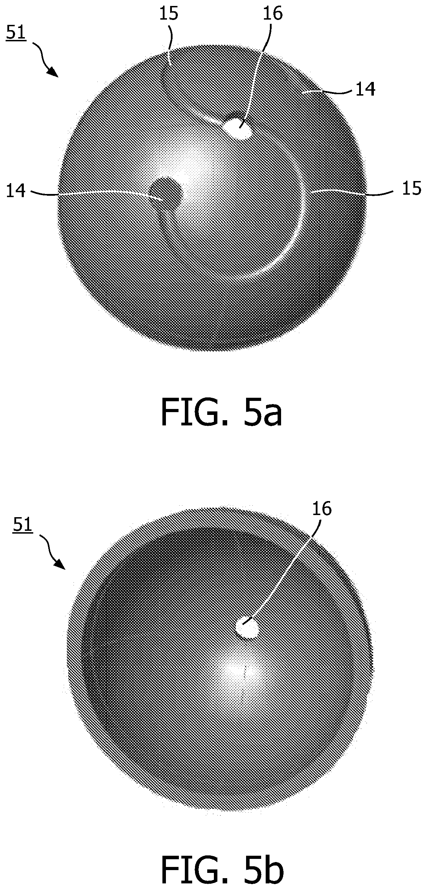

Fig. 5a and 5b show two different perspectives of a bottom part 51 of this end cap 41. This end cap 41 with the spiral channels 15 may be constructed from two pieces. The bottom piece 51 has the central hole 16 facing the drug reservoir 13. The inner wall of the exit path 15 is shown as a groove cut into the surface. In the bottom part 51, also a cut-in is provided at the positions of the dispensing holes 14.

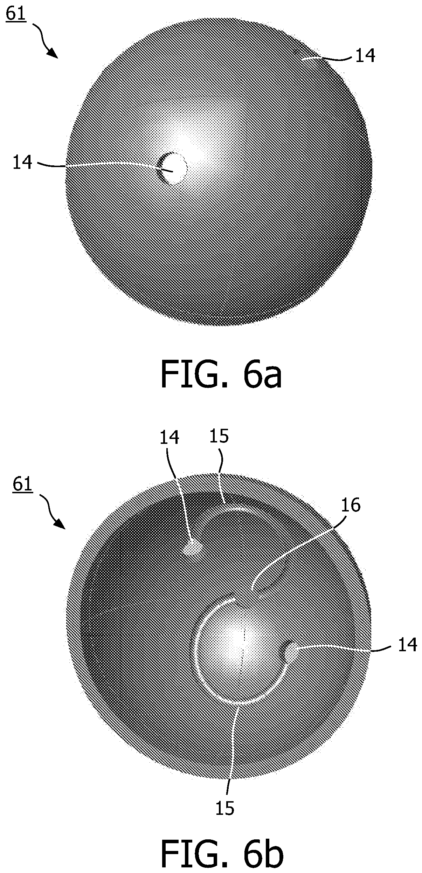

Fig. 6a and 6b show two different perspectives of a top part 61 of the end cap 41 of Fig. 4. The top piece 61 has the outer wall of the exit path 15 and the exit holes 14 to the exterior environment. In the top part 61, also a cut-in is provided at the position of the entry hole 16.

In an alternative embodiment, only the top part of the cap has a groove cut for the exit path 15 while the bottom part has a center hole only. In this embodiment, no rotational alignment is needed to assemble two parts together. The same advantageous effect is obtained by only providing a groove in the bottom part and providing a center hole in the top part.

In a further embodiment, the rotational alignment problem is solved in a different way. The bottom part of the cap has a second hole next to the center hole and the top part of the cap has a corresponding pin structure. The position and structure of both top pin and bottom second hole are so aligned that the bottom part and the top part are properly held together after the pin is inserted into the second hole. Another function of the second hole in the bottom part may be for air ventilation during loading medicine into the drug reservoir.

In preferred embodiments, the position of the entry point relative to the dispensing hole 14 is such that a possibility of drug release caused by a siphon effect is small. This may, e.g., be accomplished by designing the exit path 15 in such a way that the gravity potential between the entry point 16 and the dispensing hole 14 is always small, no matter the orientation of the pill 10. This may, e.g., be achieved by putting the entry point 16 and the dispensing hole 14 close together. A smaller gravity potential, results in a smaller siphon effect. Preferably, friction between the drug and the inner wall of the exit path 15 is large enough to impede drug release due to the siphon effect.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware.

## Claims

1. A drug delivery device (10) comprising:
a capsule,
a drug reservoir (13) in the capsule for containing a drug,
an entry point (16) in fluid communication with the drug reservoir (13) and through which the drug can exit the drug reservoir (13),
at least one dispensing hole (14) in fluid communication with the drug reservoir (13) and through which the drug can exit the capsule,
an actuator system (17) for pushing the drug from the drug reservoir (13) into the entry point (16) and through the dispensing hole (14),
at least one exit path (15) for coupling the entry point (16) with the dispensing hole (14), **characterized in that** the exit path (15) being configured such that for any possible orientation of the drug delivery device (10) a part of the exit path (15) is arranged such that gravity causes drug contained in the exit path (15) to travel in a direction away from the dispensing hole (14), toward the entry point (16), or gravity causes drug in the drug reservoir (13) to move away from the entry point (16).

2. A drug delivery device as claimed in claim 1, wherein the drug delivery device is an implantable drug delivery device.

3. A drug delivery device (10) as claimed in claim 1, wherein the drug delivery device (10) is a swallowable drug delivery device.

4. A drug delivery device (10) as claimed in any one of claims 1-3, wherein the position of the entry point (16) is close relative to the dispensing hole (14) so that a possibility of drug release caused by a siphon effect is small.

5. A drug delivery device (10) as claimed in any one of claims 1-4, wherein the exit path (15) has a spiral shape.

6. A drug delivery device (10) as claimed in any one of claims 1-5, further comprising a second dispensing hole (14) for delivering the drug to the environment of the drug delivery device (10), the second dispensing hole (14) also being coupled to the exit path (15).

7. A drug delivery device (10) as claimed in any one of claims 1-6, further comprising an end cap (41) for closing the drug reservoir (13), the exit path (15) being integrated in the end cap (41).

8. A drug delivery device (10) as claimed in claim 7, wherein the end cap (41) is constructed from a top piece (61) and a bottom piece (51), the top piece (61) comprising the dispensing hole (14) and the bottom piece (51) comprising the entry point (16), the top piece (61) and/or the bottom piece (51) comprising a groove for forming the exit path (15).

9. A drug delivery device (10) as claimed in claim 8, wherein only the top piece (61) comprises the groove and wherein the entry point (16) is positioned at a center of the bottom piece (51).

10. A drug delivery device (10) as claimed in claim 8, wherein only the bottom piece (51) comprises the groove and wherein the dispensing hole (14) is positioned at a center of the top piece (61).

11. A drug delivery device (10) as claimed in any one of claims 1-10, wherein the exit path (15) further comprises a second entry point for coupling the drug reservoir (13) to the exit path (15).

## Patentansprüche

1. Medizinabgabevorrichtung (10), umfassend:
eine Kapsel,
einen Medizinbehälter (13) in der Kapsel zur Aufnahme einer Medizin,
einen mit dem Medizinbehälter (13) in Fluidverbindung stehenden Zugangspunkt (16), durch den die Medizin den Medizinbehälter (13) verlassen kann,
zumindest eine mit dem Medizinbehälter (13) in Fluidverbindung stehende Verteilungsöffnung (14), durch die die Medizin die Kapsel verlassen kann,
ein Betätigungssystem (17) zum Befördern der Medizin aus dem Medizinbehälter (13) in den Zugangspunkt (16) und durch die Verteilungsöffnung (14),
zumindest einen Austrittsweg (15) zur Verbindung des Medizinbehälters (13) mit der Verteilungsöffnung (14), **dadurch gekennzeichnet, dass** der Austrittsweg (15) so gestaltet ist, dass bei jeder möglichen Ausrichtung der Medizinabgabevorrichtung (10) ein Teil des Austrittswegs (15) derart angeordnet ist, dass die Schwerkraft bewirkt, dass im Austrittsweg (15) enthaltene Medizin sich in Richtung von der Verteilungsöffnung (14) weg zum Zugangspunkt (16) hin bewegt, oder dass die Schwerkraft bewirkt, dass Medizin im Medizinbehälter (13) sich vom Zugangspunkt (16) weg bewegt.

2. Medizinabgabevorrichtung nach Anspruch 1, wobei die Medizinabgabevorrichtung eine implantierbare Medizinabgabevorrichtung ist.

3. Medizinabgabevorrichtung (10) nach Anspruch 1, wobei die Medizinabgabevorrichtung eine schluckbare Medizinabgabevorrichtung ist.

4. Medizinabgabevorrichtung (10) nach einem der Ansprüche 1 bis 3, wobei die Position des Zugangspunkts (16) nah bei der Verteilungsöffnung (14) ist, so dass die Möglichkeit einer durch Heberwirkung verursachten Medizinabgabe gering ist.

5. Medizinabgabevorrichtung (10) nach einem der Ansprüche 1 bis 4, wobei der Austrittsweg (15) spiralförmig ist.

6. Medizinabgabevorrichtung (10) nach einem der Ansprüche 1 bis 5, die ferner eine zweite Verteilungsöffnung (14) zur Abgabe des Medikaments in die Umgebung der Medizinabgabevorrichtung (10) aufweist, wobei auch die zweite Verteilungsöffnung (14) mit dem Austrittsweg (15) verbunden ist.

7. Medizinabgabevorrichtung (10) nach einem der Ansprüche 1 bis 6, die ferner eine Endkappe (41) zum Verschließen des Medizinbehälters (13) aufweist, wobei der Austrittsweg (15) in die Endkappe (41) integriert ist.

8. Medizinabgabevorrichtung (10) nach Anspruch 7, wobei die Endkappe (41) aus einem Oberteil (61) und einem Unterteil (51) besteht, wobei das Oberteil (61) die Verteilungsöffnung (14) umfasst und das Unterteil (51) den Zugangspunkt (16) umfasst, und wobei das Oberteil (61) und/oder das Unterteil (51) eine Nut zur Ausbildung des Austrittswegs (15) umfassen.

9. Medizinabgabevorrichtung (10) nach Anspruch 8, wobei nur das Oberteil (61) die Nut aufweist und wobei der Zugangspunkt (16) in einer Mitte des Unterteils (51) angeordnet ist.

10. Medizinabgabevorrichtung (10) nach Anspruch 8, wobei nur das Unterteil (51) die Nut aufweist und wobei die Verteilungsöffnung (14) in einer Mitte des Oberteils (61) angeordnet ist.

11. Medizinabgabevorrichtung (10) nach einem der Ansprüche 1 bis 10, wobei der Austrittsweg (15) ferner einen zweiten Zugangspunkt aufweist zur Verbindung des Medizinbehälters (13) mit dem Austrittsweg (15).

## Revendications

1. Dispositif de distribution de médicament (10) comprenant :
une capsule,
un réservoir de médicament (13) dans la capsule pour contenir un médicament,
un point d'entrée (16) en communication fluidique avec le réservoir de médicament (13) et à travers lequel le médicament peut sortir du réservoir de médicament (13),
au moins un trou de distribution (14) en communication fluidique avec le réservoir de médicament (13) et à travers lequel le médicament peut sortir de la capsule,
un système actionneur (17) pour pousser le médicament hors du réservoir à médicament (13) jusque dans le point d'entrée (16) et à travers le trou de distribution (14),
au moins un trajet de sortie (15) pour coupler le point d'entrée (16) avec le trou de distribution (14), **caractérisé en ce que** le trajet de sortie (15) est configuré de telle façon que pour toute orientation possible du dispositif de distribution de médicament (10) une partie du trajet de sortie (15) est agencé de telle façon que la gravité amène le médicament contenu dans le trajet de sortie (15) à se déplacer dans une direction en éloignement du trou de distribution (14), vers le point d'entrée (16), ou que la gravité amène le médicament dans le réservoir de médicament (13) à se déplacer en éloignement du point d'entrée (16).

2. Dispositif de distribution de médicament selon la revendication 1, dans lequel le dispositif de distribution de médicament est un dispositif de distribution de médicament implantable.

3. Dispositif de distribution de médicament (10) selon la revendication 1, dans lequel le dispositif de distribution de médicament (10) est un dispositif de distribution de médicament susceptible d'être avalé.

4. Dispositif de distribution de médicament (10) selon l'une quelconque des revendications 1 à 3, dans lequel la position du point d'entrée (16) est proche par rapport au trou de distribution (14), de sorte qu'une possibilité d'une libération de médicament provoquée par un effet siphon est faible.

5. Dispositif de distribution de médicament (10) selon l'une quelconque des revendications 1 à 4, dans lequel le trajet de sortie (15) a une forme en spirale.

6. Dispositif de distribution de médicament (10) selon l'une quelconque des revendications 1 à 5, comprenant en outre un second trou de distribution (14) pour distribuer le médicament vers l'environnement du dispositif de distribution de médicament (10), le second trou de distribution (14) étant également couplé au trajet de sortie (15).

7. Dispositif de distribution de médicament (10) selon l'une quelconque des revendications 1 à 6, comprenant en outre un capuchon d'extrémité (41) pour fermer le réservoir de médicament (13), le trajet de sortie (15) étant intégré dans le capuchon d'extrémité (41).

8. Dispositif de distribution de médicament (10) selon la revendication 7, dans lequel le capuchon d'extrémité (41) est réalisé d'une pièce supérieure (61) et d'une pièce inférieure (51), la pièce supérieure (61) comprenant le trou de distribution (14) et la pièce inférieure (51) comprenant le point d'entrée (16), la pièce supérieure (61) et/ou la pièce inférieure (51) comprenant une rainure pour former le trajet de sortie (15).

9. Dispositif de distribution de médicament (10) selon la revendication 8, dans lequel uniquement la pièce supérieure (61) comprend la rainure, et dans lequel le point d'entrée (16) est positionné à un centre de la pièce inférieure (51).

10. Dispositif de distribution de médicament (10) selon la revendication 8, dans lequel uniquement la pièce inférieure (51) comprend la rainure et dans lequel le trou de distribution (14) est positionné à un centre de la pièce supérieure (61).

11. Dispositif de distribution de médicament (10) selon l'une quelconque des revendications 1 à 10, dans lequel le trajet de sortie (15) comprend un second point d'entrée pour coupler le réservoir de médicament (13) au trajet de sortie (15).
